# EUROPÄISCHE PATENTSCHRIFT

(11) **EP 0 731 086 B1**
(45) Veröffentlichungstag und Bekanntmachung des Hinweises auf die Patenterteilung: **28.04.1999**
(21) Anmeldenummer: 96810115.4
(22) Anmeldetag: 01.03.1996
(51) Int. Cl.: C07C 253/00, C07C 255/49

(54) **Verfahren zur Herstellung aromatischer Nitrile**
Process for the preparation of aromatic nitriles
Procédé de préparation de nitriles aromatiques

(30) Priorität: 09.03.1995 CH 687/95
(43) Veröffentlichungstag der Anmeldung: 11.09.1996
(73) Patentinhaber: Ciba Specialty Chemicals Holding Inc., 4057 Basel (CH)
(72) Erfinder: Kudschus, Martin, Dr., 1762 Givisiez (CH)

(56) Entgegenhaltungen:
- EP-A- 0 080 700
- EP-A- 0 144 885
- EP-A- 0 550 762
- EP-A- 0 609 179
- DATABASE WPI Week 7045 Derwent Publications Ltd., London, GB; AN 70-83813r XP002004623 & JP-B-45 035 771 (MITSUI PETROCHEM INDS LTD)

## Beschreibung

Die vorliegende Anmeldung bettrifft ein Verfahren zur Herstellung von aromatischen Nitrilen durch lösungsmittelfreie Umsetzung entsprechender Aldehyde mit einem Hydroxylammonium-Salz und einem anorganischen, wasserfreien Sulfat.

Die Umsetzung von Aldehyden mit Hydroxylammoniumsalzen und die anschliessende Dehydratisierung des erhaltenen Oxims zum Nitril sind seit langem bekannt. Zur Dehydratisierung wurden verschiedene Methoden vorgeschlagen, so in C.A. 85, 93176e (1976) durch Erhitzen in Dimethylformamid, in EP 080700 durch azeotropes Abdestillieren des Wassers mit Hilfe eines ein azeotropes Gemisch bildenden und mit Wasser nicht mischbaren Lösungsmittels, in Synthesis 1979/2,112-113 (1979) und in Huaxue Shiji 12(5), 314, 292 (1990) durch Erhitzen in Ameisensäure, in Joumal of Nanjing Univ., 26/2, 263-266 (1990), durch Erhitzen in Ameisensäure oder Eisessig, in J. Chem. Soc. IX, 43 (1933) durch Erhitzen in Essigsäureanhydrid und in EP 609179 durch Erhitzen in Propionsäure.

In EP 609179 wird in einer Reaktionsgleichung postuliert, dass bei der Reaktion eines Benzaldehydes mit Hydroxylammoniumsulfat und Natriumpropionat in Propionsäure Natriumsulfat entsteht, doch ist dabei entscheidend, dass Propionsäure als Lösungsmittel verwendet wird. Zudem steht dieses Postulat im Widerspruch mit der offenbarten Stöchiometrie, die zu Natriumhydrogensulfat führt.

Von einer Verwendung von Lösungsmitteln im grosstechnischen Massstab sieht man aber heutzutage aus umweltpolitischen und ökonomischen Überlegungen wenn möglich ab, so dass Wege zur lösungsmittelfreien Herstellung von Nitrilen wünschenswert sind. Die Herstellung von Oximen gelingt lösungsmittelfrei durch die Behandlung eines Aldehydes und Hydroxylammoniumchlorid mit Mikrowellen in Gegenwart von auf Aluminiumoxid adsorbiertem Kaliumfluorid [Chemistry & Industry 1991/5, 176 (1991)], jedoch verlangt die weitere Reaktion zum Nitril den Zusatz von Schwefelkohlenstoff und Acetonitril. Ohne Lösungsmittel können Nitrile zwar durch Behandlung mit Mikrowellen oder Bestrahlung mit Infrarotlicht erhalten werden, aber die Reaktanden müssen dabei auf mexikanischem Bentonit adsorbiert sein [Synthetic Communications 22/14, 2125-2128 (1992)].

Es hat sich herausgestellt, dass einige dieser bekannten Methoden nur zu unbefriedigende Ausbeuten führen, und dass die Reinheit der nach den anderen bekannten Methoden hergestellten Benzonitrile insbesondere im Hinblick auf die Herstellung hochwertiger Diketopyrrolopyrrolpigmente noch einiges zu wünschen übrig lässt. Insbesondere ergeben viele Aldehyde bei der obenerwähnten Bentonit-Methode offenbar strukturabhängig recht kärgliche Ausbeuten.

Es ist nun ganz überraschenderweise gefunden worden, dass dieselben Nitrile in ausgezeichneter Reinheit erhalten werden können, wenn man auf ein Lösungsmittel verzichtet und einfach das Reaktionsgemisch aus Aldehyd und Hydroxylammoniumsalz zusammen mit einem wasserfreien anorganischen Sulfat erhitzt. Die Ausbeute ist unerwartet etwa gleich hoch wie bei der Verwendung von Propionsäure als Lösungsmittel (EP 609179), und sehr überraschend viel höher als bei der Verwendung einer Tonerde unter lösungsmittelfreien Bedingungen. Dadurch ist als weiterer Vorteil eine höhere Volumenausbeute möglich.

Dies ist umso erstaunlicher, als die wasserfreien anorganischen Sulfate, welche die vorliegende Erfindung charakterisieren, weder sauer reagieren noch eine aktive Oberfläche oder ein besonderes Adsorptionsvermögen gegenüber organischen Molekülen aufweisen.

Ausschlaggebend für die überraschend guten Resultate der vorliegenden Erfindung ist die Tatsache, dass unter diesen Bedingungen eine präzise Reaktionsführung sowie kürzere Reaktionszeiten erreichbar sind. Durch Wahl günstiger, verschieden hoher Temperaturen für die Reaktion zum Oxim, beziehungsweise für die nachfolgende Dehydratisierung zum Nitril, werden schnelle und sehr selektive Reaktionen gefördert, so dass die Bildung von Nebenprodukten, insbesondere die Bildung des unerwünschten Carbonamides, weitgehend verhindert wird.

Die vorliegende Anmeldung betrifft demnach ein Verfahren zur Herstellung eines Nitrils der Formel (I) 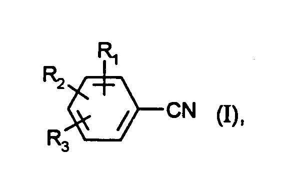 worin
R₁, R₂ und R₃ unabhängig voneinander Wasserstoff, Halogen, Cyano, Hydroxy, Carboxy, C₁-C₁₈-Alkyl, C₁-C₁₈-Alkoxy, C₁-C₁₈-Alkylthio, C₁-C₁₈-Alkylsulfonyl, C₁-C₁₈-Alkylamino, Di(C₁-C₁₈-alkyl)amino, R₄-C₆-C₁₀-Aryl, R₄-C₆-C₁₀-Aryloxy, R₄-C₆-C₁₀-Arylthio, R₄-C₆-C₁₀-Arylsulfonyl oder R₄-C₆-C₁₀-Arylamino bedeuten,
oder, falls R₂ und R₃ ortho zueinander liegen, R₂ und R₃ zusammen eine gesättigte oder ein- oder zweimal ungesättigte, mit einem Rest R₄ substituierte 4-gliedrige Kohlenstoffbrücke bilden,
und R₄ Wasserstoff, Halogen, Cyano, Hydroxy, Carboxy, C₁-C₁₈-Alkyl, C₁-C₁₈-Alkoxy, C₁-C₁₈-Alkylthio, C₁-C₁₈-Alkylsulfonyl, C₁-C₁₈-Alkylamino oder Di(C₁-C₁₈-alkyl)amino ist,
durch Umsetzung eines Aldehyds der Formel (II) 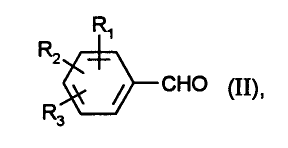 worin R₁, R₂ und R₃ die oben angegebene Bedeutung haben, mit einem Hydroxylammoniumsalz, und nachfolgende Dehydratisierung durch Erhitzen auf eine erhöhte Temperatur, dadurch gekennzeichnet, dass die Umsetzung in Gegenwart eines wasserfreien anorganischen Sulfates und in Abwesenheit von Verdünnungsmitteln aus der Gruppe bestehend aus Carbonsäuren, stark polaren aprotischen Lösungsmitteln, Schwefelverbindungen und heteroaromatischen basischen Stickstoffverbindungen, stattfindet.

Zweckmässig sind wasserfreie anorganische Sulfate, welche beim Ansäuem stabile Bisulfate ergeben. Beispielsweise kann Natrium-, Kalium-, Lithium- oder Ammoniumsulfat verwendet werden, wobei Natriumsulfat bevorzugt ist.

Beim Hydroxylammoniumsalz handelt es sich beispielsweise um Hydroxylammoniumchlorid oder -sulfat, bevorzugt um Hydroxylammoniumsulfat.

Halogen ist beispielsweise Chlor, Brom oder Fluor.

C₁-C₈-Alkyl, selbst oder als Bestandteil von C₁-C₁₈-Alkyl, C₁-C₁₈-Alkoxy, C₁-C₁₈-Alkylthio, C₁-C₁₈-Alkylsulfonyl, C₁-C₁₈-Alkylamino oder Di(C₁-C₁₈-alkyl)amino, ist beispielweise C₁-C₄-Alkyl, wie Methyl, Ethyl, n-Propyl, Isopropyl, n-Butyl, sec.-Butyl, tert.-Butyl oder Isobutyl, oder n-Pentyl, n-Hexyl, n-Octyl, 2,2-Dimethylpropyl oder 1,1,3,3-Tetramethylbutyl.

C₆-C₁₀-Aryl, selbst oder als Bestandteil von R₄-C₆-C₁₀-Aryl, R₄-C₆-C₁₀-Aryloxy, R₄-C₆-C₁₀-Arylthio, R₄-C₆-C₁₀-Arylsulfonyl oder R₄-C₆-C₁₀-Arylamino, ist Phenyl oder Naphtyl.

Da beim vorliegenden Verfahren die organischen Bestandteile zweckmässig in flüssiger Form vorliegen sollten, ist es möglich, aus aromatischen Aldehyden mit Schmelzpunkt ≤ 125°C aromatische Nitrile mit Schmelzpunkt ≤ 170°C und Siedepunkt ≤ 170°C / 1 mbar völlig lösungsmittelfrei herzustellen.

Es ist aber auch möglich, inerte Verdünnungsmittel, ausgenommen Carbonsäuren, heteroaromatische basische Stickstoffverbindungen, Schwefelverbindungen und stark polare Verbindungen, in kleineren Mengen zuzusetzen. Dadurch können beispielsweise die Viskosität oder die Verteilung verbessert, das Schmelzen des Ausgangs-Aldehydes gefördert oder die Ausfällung des entstehenden Nitrils verhindert werden, so dass die gewünschten Umsetzungen zum Oxim und/oder zum Nitril begünstigt werden.

Bei den inerten Verdünnungsmitteln handelt es sich gegebenenfalls um hochsiedende Flüssigkeiten oder Festkörper mit Schmelzpunkt ≤ 70°C niedriger Polarität (Dipolmoment µ ≤ 2×10⁻¹⁸ esu), beispielsweise um Kohlenwasserstoffe, Ether, Alkohole oder Gemische davon, wie Benzinfraktionen, Petroleum, niederschmelzende Wachse, Shell-Sol®, Solvesso®, Dowtherm®, Trichlorbenzol, Diethylenglykoldimethylether, n-Octanol oder Ethylbenzoat, welche einen Siedepunkt ≥ 150°C / 1 bar, bevorzugt einen Siedepunkt ≥ 150°C/5 mbar haben.

Als inert werden solche Verdünnungsmittel betrachtet, die während der Reaktion völlig unverändert bleiben, oder deren Folgeprodukte höchstens zu Spuren von Verunreinigungen im Endprodukt (d.h. ≤ 1 Gew.-%, bezogen auf den Nitril) führen.

Carbonsäuren, heteroaromatische basische Stickstoffverbindungen, Schwefelverbindungen und stark polare Verbindungen (Dipolmoment µ > 2×10⁻¹⁸ esu), wie beispielsweise Propionsäure, Essigsäure, Ameisensäure, Pyridin, Chinolin, Schwefelkohlenstoff, Dimethylsulfoxid und Dimethylformamid, üben einen unerwünschten Einfluss auf den Reaktionsverlauf oder stören bei der Aufarbeitung und sind deshalb als Verdünnungsmittel nicht geeignet.

Bei den Aldehyden der Formel (II) handelt es sich um bekannte Verbindungen, wie zum Beispiel Benzaldehyd, 2-, 3- oder 4-Chlor-benzaldehyd, 2- oder 3-Methoxy-benzaldehyd, Anisaldehyd, 2-, 3- oder 4-Tolualdehyd, 4-tert.-Butyl-benzaldehyd, Biphenyl-4-carbaldehyd, Mesitylen-2-carbaldehyd, 1- oder 2-Naphthaldehyd, 3- oder 4-Phenoxybenzaldehyd oder Tetralin-2-carbaldehyd. Sollte es sich bei einigen Aldehyden der Formel (II) um unbekannte Verbindungen handeln, so können sie nach bekannten Methoden hergestellt werden.

Bevorzugt ist die Herstellung aromatischer Nitrile der Formel (I) mit einem Siedepunkt ≤ 170°C/1 mbar aus Aldehyden der Formel (II) mit einem Schmelzpunkt ≤ 125°C.

Ebenfalls bevorzugt ist die Herstellung aromatischer Nitrile der Formel (I), worin
R₁, R₂ und R₃ unabhängig voneinander Wasserstoff, Halogen, Cyano, Hydroxy, Carboxy, C₁-C₈-Alkyl, C₁-C₈-Alkoxy, C₁-C₈-Alkylthio, C₁-C₈-Alkylamino, Di(C₁-C₈-alkyl)amino, R₄-C₆-C₁₀-Aryl, R₄-C₆-C₁₀-Aryloxy, R₄-C₆-C₁₀-Arylthio oder R₄-C₆-C₁₀-Arylamino bedeuten,
oder, falls R₂ und R₃ ortho zueinander liegen, R₂ und R₃ zusammen eine gesättigte oder ein- oder zweimal ungesättigte, mit einem Rest R₄ substituierte 4-gliedrige Kohlenstoffbrücke bilden,
und R₄ Wasserstoff, Halogen, Cyano, Hydroxy, Carboxy, C₁-C₈-Alkyl, C₁-C₈-Alkoxy, C₁-C₈-Alkylthio, C₁-C₈-Alkylamino oder Di(C₁-C₈-alkyl)amino ist.

Besonders bevorzugt ist die Herstellung aromatischer Nitrile der Formel (I), worin
R₁, R₂ und R₃ unabhängig voneinander Wasserstoff, Halogen, C₁-C₄-Alkyl, C₁-C₄-Alkoxy, Di(C₁-C₄-alkyl)amino, R₄-C₆-C₁₀-Aryl, R₄-C₆-C₁₀-Aryloxy oder R₄-Di(C₆-C₁₀-aryl)amino, und R₄ Wasserstoff, Halogen, C₁-C₂-Alkyl, C₁-C₂-Alkoxy oder Di(C₁-C₂-alkyl)amino bedeuten,
insbesondere die Herstellung aromatischer Nitrile der Formel (I), worin
R₁ Wasserstoff, Chlor, Methyl, Methoxy, tert.-Butyl oder Phenyl, ganz besonders bevorzugt 4-Methyl, 4-tert.-Butyl oder 4-Phenyl, insbesondere 4-Phenyl, R₂ und R₃ entweder je Wasserstoff, oder beide zusammen ω-Buta-1,3-dienylen, und R4 Wasserstoff bedeuten.

Das Hydroxylammoniumsalz wird vorzugsweise mindestens in stöchiometrischer Menge, besonders bevorzugt mit leichtem Überschuss, d.h. 0,505 bis 0,58 Mol (NH₂OH)₂ · H₂SO₄ auf 1 Mol Aldehyd, eingesetzt.

Das wasserfreie Sulfat wird zweckmässig in einer Menge von 1 bis 20 Mol, bevorzugt 2 bis 12 Mol, je bezogen auf 1 Mol Aldehyd, eingesetzt.

In einer bevorzugten Ausführungsform der Erfindung, welche erstaunlicherweise besonders gut reproduzierbare, hohe Ausbeuten ergibt, wird ein wasserfreies Sulfat verwendet, dessen Anteile mit einer Komgrösse ≤ 50 µm mindestens 1 Gew.-% der Gesamtmenge wasserfreien Sulfats darstellen. Besonders bevorzugt enthält das wasserfreies Sulfat von 2 bis 20 Gew.-%, ganz besonders bevorzugt von 5 bis 15 Gew.-%, insbesondere zirka 10 Gew.-%, Anteile mit einer Komgrösse ≤ 50 µm. Der Anteil mit Komgrösse ≤ 50 µm kann durch Sieben des wasserfreien Sulfats durch einen 50 µm Sieb bestimmt werden.

Wasserfreies Sulfat mit 10 Gew.-% Anteilen mit einer Komgrösse ≤ 50 µm kann zum Beispiel derart hergestellt werden, dass 90 Gew.-Teile eines relativ grobkömigem wasserfreien Sulfats (beispielsweise Siebrückstand im 50 µm Sieb), mit 10 Gew.-Teilen eines relativ feinkömigen wasserfreien Sulfats (beispielsweise gemahlen und durch einen 50 µm Sieb gesiebt), miteinander eng vermischt werden.

Verdünnungsmittel werden gegebenenfalls in Mengen bis 150 Gew.-%, bevorzugt bis 50 Gew.-%, bezogen auf den eingesetzten Aldehyd, zugegeben.

Die optimale Temperaturführung bei der Erhitzung des Reaktionsgemisches hängt vom jeweiligen Aldehyd ab. Zweckmässig erhitzt man in zwei Stufen innerhalb 1-5 Stunden von 25°C zunächst auf 50-120°C, dann auf 100-200°C, wobei bei der ersten Temperatur hauptsächlich das Oxim gebildet und die Schwefelsäure vom Sulfat gebunden werden, und bei der zweiten, höheren Temperatur das Nitril vollständig entsteht und gebildetes Reaktionswasser abdestilliert wird.

Bevorzugt sind möglichst kurze Reaktionszeiten bei der zweiten, höheren Temperatur. Zweckmässig ist es deshalb, die Reaktion unter vermindertem Druck, bevorzugt bei 2-100 mbar, besonders bevorzugt bei 5-50 mbar, durchzuführen, wobei gebildetes Reaktionswasser besonders schnell abdestilliert werden kann.

Die Aufarbeitung kann zum Beispiel konventionell geschehen, indem das Rohprodukt, gegebenenfalls mit Hilfe eines organischen Lösungsmittels, vom unlöslichen Salz getrennt wird, oder indem die anorganischen Bestandteile in Wasser aufgelöst und die wässrige Phase abgetrennt werden. Das Reinprodukt kann danach aus dem Rohprodukt beispielsweise durch Kristallisation, Destillation oder einer anderen der vielen dem Fachmann wohlbekannten Methoden gewonnen werden.

Geschieht die Aufarbeitung konventionell, so verwendet man vorzugsweise ein inertes organisches Lösungsmittel mit einem tiefen Siedepunkt (∼40-120°C), wie beispielsweise Aceton, Methylethylketon, Methanol, Essigsäureethylester oder Toluol. Bevorzugt sind Toluol und insbesondere Methanol.

Die bevorzugte Aufarbeitung ist jedoch die direkte Destillation des reinen Produktes aus dem Reaktionsgemisch, unmittelbar nach Beendigung der Dehydratisierung, durch Erhöhung der Temperatur und/oder Verminderung des Druckes. Dadurch können Nitrile in ganz besonders hoher Reinheit erhalten werden.

Geschieht die Aufarbeitung destillativ, so kann durch geeignete Wahl der Destillationsbedingungen der Reinheitsgrad des Reinproduktes nochmals gesteigert werden, indem zum Beispiel eine Destillationskolonne mit mehreren theoretischen Böden und ein hohes Rücklaufverhältnis am Kopf gewählt werden. Dem Fachmann ist die Einstellung der gewünschten Qualität durch Beeinflussung der Destillationsparameter bestens bekannt.

Im allgemeinen ist es jedoch überflüssig, spezielle Massnahmen bei der Destillation des Produktes zu treffen, da die Reinheit der nach der vorliegenden Erfindung hergestellten Produkte schon überraschend ausserordentlich hoch ist.

Fallen bei der Destillation Mischfraktionen an, welche im Wesentlichen aus Aldehyd und Nitril bestehen, so können diese Mischfraktionen im Verhältnis zum Anteil des darin enthaltenen Aldehyds vorteilhaft als Edukt für das erfindungsgemässe Verfahren wiederverwendet werden. Durch die Möglichkeit der Rezyklisierung des nicht umgesetzten Aldehyds weist das erfindungsgemässe Verfahren somit einen weiteren Vorteil auf.

Die durch das erfindungsgemässe Verfahren erhältlichen Nitrile sind wertvolle Zwischenprodukte u.a. für die Herstellung von 1,4-Diketo-2,5-dihydro-3,6-diaryl-pyrrolo[4,3-c]pyrrolpigmenten, wofür sie sich infolge ihrer hohen Reinheit ganz besonders gut eignen. Die Herstellung von Diketopyrrolopyrrolpigmenten ausgehend von Nitrilen ist wohlbekannt und beispielsweise in US 4,579,949 beschrieben.

Die nachfolgenden Beispiele erläutern die Erfindung:

Beispiel 1: Ein Schaufelradtrockner wird unter Rühren (20 U/Min) bei Raumtemperatur mit 1007 Gew.-Teilen wasserfreiem Natriumsulfat, 222 Gew.-Teilen Biphenyl-4-carbaldehyd und 105 Gew.-Teilen Hydroxylammoniumsulfat beschickt. Nach Evakuierung auf 13 mbar wird auf 70°C erhitzt und 3 Stunden bei dieser Temperatur nachgerührt. Anschliessend wird auf 130°C erhitzt, wobei ca. 43 Gew.-Teile Wasser abdestilliert werden. Nach einer halben Stunde wird der Druck auf 4 mbar reduziert und die Manteltemperatur innerhalb 60-70 Min. sukzessive auf 170°C erhöht, wobei das Produkt fraktioniert destilliert wird. Man erhält 194 Gew.-Teile Biphenyl-4-carbonitril mit einer Reinheit von ∼98-99% (HPLC) und einem Schmelzpunkt von 83-85°C nebst 10 Gew.-Teilen einer weniger reinen Fraktion.

Beispiel 2: Ein Schaufelradtrockner wird unter Rühren (20 U/Min) bei Raumtemperatur mit 1800 Gew.-Teilen wasserfreiem Natriumsulfat, 365 Gew.-Teilen 4-tert.-Butylbenzaldehyd und 194 Gew.-Teilen Hydroxylammoniumsulfat beschickt. Nach Evakuierung auf 25 mbar wird auf 100°C erhitzt und 2 Stunden bei dieser Temperatur nachgerührt. Anschliessend wird für 40 Min. auf 125°C erhitzt, wobei ca. 82 Gew.-Teile Wasser abdestilliert werden. Die Destillation des Nitrils wird dann durch sukzessive Absenkung des Druckes auf 10 mbar und Erhöhung der Manteltemperatur auf 170°C eingeleitet und gesteuert. Man erhält 300 Gew.-Teile 4-tert.-Butylbenzonitril mit einer Reinheit von ∼98-99% (HPLC) nebst 18 Gew.-Teilen einer weniger reinen Fraktion.

Beispiel 3: Ein Schaufelradtrockner wird unter Rühren (20 U/Min) bei Raumtemperatur mit 8000 Gew.-Teilen wasserfreiem Natriumsulfat, 1602 Gew.-Teilen 4-Methylbenzaldehyd und 1149 Gew.-Teilen Hydroxylammoniumsulfat beschickt. Nach Evakuierung auf 35 mbar wird auf 80°C erhitzt und 2½ Stunden bei dieser Temperatur nachgerührt. Anschliessend wird bei einem Druck von 80 mbar auf 125°C erhitzt und 10 min gehalten; dann wird der Druck auf 60 mbar abgesenkt und weitere 20 min gehalten, wobei insgesamt ca. 480 Gew.-Teile Wasser abdestilliert werden. Die Destillation des Nitrils wird durch sukzessive Druckabsenkung bis auf 10 mbar und nachfolgende Erhöhung der Manteltemperatur bis auf 170°C eingeleitet und gesteuert.
Man erhält 1265 Gew.-Teile 4-Methylbenzonitril mit einer Reinheit von über 98% (HPLC), sowie 37 Gewichtsteile einer Mischfraktion, welche im Wesentlichen aus 4-Methylbenzonitril und 4-Methylbenzaldehyd besteht.

Beispiel 4: Ein Schaufelradtrockner wird unter Rühren (20 U/Min) bei Raumtemperatur mit 4000 Gew.-Teilen wasserfreiem Natriumsulfat, 951 Gew.-Teilen Vanillin und 538,6 Gew.-Teilen Hydroxylammoniumsulfat beschickt. Nach Evakuierung auf ca. 10 mbar wird auf 85°C erhitzt und 3 Stunden bei dieser Temperatur nachgerührt. Anschliessend wird bei gleichem Druck auf 130°C erhitzt und 25 min bei dieser Temperatur gehalten. Während der gesamten Reaktionsphase werden insgesamt ca. 250 Gew.-Teile Wasser abdestilliert. Die Destillation des Nitrils wird durch Druckabsenkung auf ca. 5 mbar und nachfolgende sukzessive Erhöhung der Manteltemperatur bis auf 200°C eingeleitet und gesteuert. Man erhält 693 Gew.-Teile Vanillinsäurenitril mit einer Reinheit von über 98% (Dünnschichtschromatographie) sowie 43,5 Gew.-Teile einer Mischfraktion, welche im Wesentlichen aus Vanillinsäurenitril und Vanillin besteht. Der Schmelzpunkt des erhaltenen Vanillinsäurenitrils beträgt 87,5-89,5°C.

Beispiel 5: Ein Schaufelradtrockner wird unter Rühren (20 U/Min) bei Raumtemperatur mit 4000 Gew.-Teilen wasserfreiem Natriumsulfat, 951 Gew.-Teilen Vanillin und 538,6 Gew.-Teilen Hydroxylammoniumsulfat beschickt. Nach Evakuierung auf ca. 10 mbar wird auf 85°C erhitzt und 3 Stunden bei dieser Temperatur nachgerührt. Anschliessend wird bei gleichem Druck auf 130°C erhitzt und 25 min bei dieser Temperatur gehalten. Während der gesamten Reaktionsphase werden insgesamt ca. 250 Gew.-Teile Wasser abdestilliert. Die Reaktionsmasse wird auf 100°C abgekühlt und der Unterdruck mit Stickstoff aufgehoben. Man versetzt mit 3000 Gew.-Teilen Toluol, hält weitere 20 min bei 100°C unter Stickstoff und lässt unter Rühren auf Raumtemperatur abkühlen. Die Suspension wird filtriert, der Filterkuchen dreimal mit je 3000 Gew.-Teilen Toluol heiss extrahiert und die Mutterlauge zusammen mit den vereinigten Toluolextrakten zur Trockene eingedampft. Der Rückstand (ca. 875 Gew.-Teile) wird in 5500 Gew.-Teilen Methylenchlorid aufgenommen und über 2100 Gew.-Teilen Kieselgel 60 [70 - 230 mesh (Merck)] säulenchromatographiert, wobei zur Eluierung ca. 33000 Gew.-Teile Methylenchlorid verwendet werden. Die nach Verwerfen des Säulentotlaufes übergehenden ersten ca. 30000 Gew.-Teile Eluat werden vereinigt und zur Trockene eingedampft.
Man erhält 719 Gew.-Teile Vanillinsäurenitril mit einer Reinheit von über 98% (Dünnschichtschromatographie) und einem Schmelzpunkt von 88-90°C.

Beispiel 6: Ein Schaufelradtrockner wird unter Rühren (20 U/Min) bei Raumtemperatur mit 4000 Gew.-Teilen wasserfreiem Natriumsulfat, 950 Gew.-Teilen 4-(Methylthio)benzaldehyd und 538,4 Gew.-Teilen Hydroxylammoniumsulfat beschickt. Nach Evakuierung auf 25 mbar wird auf 80°C erhitzt und 3 Stunden bei dieser Temperatur gehalten. Anschliessend wird bei gleichem Druck auf 130°C erhitzt und 30 min bei dieser Temperatur nachgerührt. Während der gesamten Reaktionsphase werden insgesamt ca. 240 Gew.-Teile Wasser abdestilliert. Die Destillation des Nitrils wird durch Druckabsenkung auf 5 mbar und nachfolgende sukzessive Erhöhung der Manteltemperatur bis auf 150°C eingeleitet und gesteuert. Man erhält 708 Gew.-Teile 4-(Methylthio)benzonitril mit einer Reinheit von mindestens 98% (Dünnschichtschromatographie) sowie 18 Gew.-Teile einer Mischfraktion, welche im Wesentlichen aus 4-(Methylthio)benzonitril und 4-(Methylthio)benzaldehyd besteht. Der Schmelzpunkt des erhaltenen 4-(Methylthio)benzonitrils beträgt 61-63°C.

Beispiel 7: Ein Schaufelradtrockner wird unter Rühren (20 U/Min) bei Raumtemperatur mit 1280 Gew.-Teilen wasserfreiem Natriumsulfat, 462 Gew.-Teilen 3-Brom-4-hydroxy-5-methoxybenzaldehyd, 172 Gew.-Teilen Hydroxylammoniumsulfat und 298 Gew.-Teilen 1-Chlornaphthalin beschickt. Nach Evakuierung auf ca. 70 mbar wird auf 130°C erhitzt und 3½ Stunden bei dieser Temperatur gehalten. Die Reaktionsmasse wird anschliessend auf 30°C abgekühlt und der Unterdruck mit Stickstoff mit Stickstoff aufgehoben. Man versetzt mit 2650 Gew.-Teilen Methylenchlorid, rührt 20 Min. bei Raumtemperatur und filtriert die Suspension ab. Der Filterkuchen wird dreimal mit je 2000 Gew.-Teilen Methylenchlorid extrahiert und die Mutterlauge zusammen mit den vereinigten Methylenchloridextrakten auf ¹/₃ des ursprünglichen Volumens eingedampft. Das in Methylenchlorid gelöste Rohprodukt wird über 1400 Gew.-Teilen Kieselgel 60 [70 - 230 mesh (Merck)] säulenchromatographiert, wobei zur Eluierung ca. 26500 Gew.-Teile Methylenchlorid verwendet werden. Die nach Verwerfen des Säulentotlaufes übergehenden ersten ca. 17000 Gew.-Teile Eluat werden vereinigt und eingedampft. Der Rückstand wird bei ca. 80°C (im Vakuum) von anhaftenden Lösungsmittelresten (1-Chlomaphthalin) befreit.
Man erhält 334,5 Gew.-Teile 3-Brom-4-hydroxy-5-methoxybenzonitril mit einer Reinheit von mindestens 98% (Dünnschichtschromatographie) und einem Schmelzpunkt von 144-145°C.

Beispiel 8: Ein Schaufelradtrockner wird unter Rühren (20 U/Min) bei Raumtemperatur mit 1280 Gew.-Teilen wasserfreiem Natriumsulfat, 462 Gew.-Teilen 3-Brom-4-hydroxy-5-methoxybenzaldehyd und 172 Gew.-Teilen Hydroxylammoniumsulfat beschickt. Nach Evakuierung auf ca. 25 mbar wird auf 130°C erhitzt und 3½ Stunden bei dieser Temperatur gehalten. Die Reaktionsmasse wird anschliessend auf ca. 30°C abgekühlt und der Unterdruck mit Stickstoff aufgehoben. Man versetzt mit 2650 Gew.-Teilen Methylenchlorid, rührt 20 Min. bei Raumtemperatur und filtriert die Suspension ab. Der Filterkuchen wird dreimal mit je 2000 Gew.-Teilen Methylenchlorid extrahiert und die Mutterlauge zusammen mit den vereinigten Methylenchloridextrakten auf ¹/₃ des ursprünglichen Volumens eingedampft. Das in Methylenchlorid gelöste Rohprodukt wird über 1400 Gew.-Teilen Kieselgel 60 [70 - 230 mesh (Merck)] säulenchromatographiert, wobei zur Eluierung ca. 26500 Gew.-Teile Methylenchlorid verwendet werden. Die nach Verwerfen des Säulentotlaufes übergehenden ersten ca. 17000 Gew.-Teile Eluat werden vereinigt und eingedampft. Der Rückstand wird 80°C getrocknet. Man erhält 281 Gew.-Teile 3-Brom-4-hydroxy-5-methoxybenzonitril mit einer Reinheit von ca. 95 % (Dünnschichtschromatographie / Rest = 3-Brom-4-hydroxy-5-methoxybenzaldehyd) und einem Schmelzpunkt von 139-142°C.

## Patentansprüche

1. Verfahren zur Herstellung eines Nitrils der Formel (I) 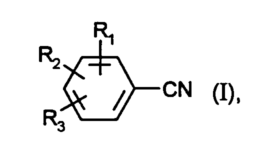
worin R₁, R₂ und R₃ unabhängig voneinander Wasserstoff, Halogen, Cyano, Hydroxy, Carboxy, C₁-C₁₈-Alkyl, C₁-C₁₈-Alkoxy, C₁-C₁₈-Alkylthio, C₁-C₁₈-Alkylsulfonyl, C₁-C₁₈-Alkylamino, Di(C₁-C₁₈-alkyl)amino, R₄-C₆-C₁₀-Aryl, R₄-C₆-C₁₀-Aryloxy, R₄-C₆-C₁₀-Arylthio, R₄-C₆-C₁₀-Arylsulfonyl oder R₄-C₆-C₁₀-Arylamino bedeuten,
oder, falls R₂ und R₃ ortho zueinander liegen, R₂ und R₃ zusammen eine gesättigte oder ein- oder zweimal ungesättigte, mit einem Rest R₄ substituierte 4-gliedrige Kohlenstoffbrücke bilden,
und R₄ Wasserstoff, Halogen, Cyano, Hydroxy, Carboxy, C₁-C₁₈-Alkyl, C₁-C₁₈-Alkoxy, C₁-C₁₈-Alkylthio, C₁-C₁₈-Alkylsulfonyl, C₁-C₁₈-Alkylamino oder Di(C₁-C₁₈-alkyl)amino ist,
durch Umsetzung eines Aldehyds der Formel (II) 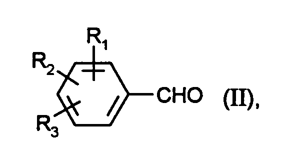 worin R₁, R₂ und R₃ die oben angegebene Bedeutung haben, mit einem Hydroxylammoniumsalz, und nachfolgende Dehydratisierung durch Erhitzen auf eine erhöhte Temperatur, dadurch gekennzeichnet, dass die Umsetzung in Gegenwart eines wasserfreien anorganischen Sulfates und in Abwesenheit von Verdünnungsmitteln aus der Gruppe bestehend aus Carbonsäuren, stark polaren aprotischen Lösungsmitteln, Schwefelverbindungen und heteroaromatischen basischen Stickstoffverbindungen, stattfindet.

2. Verfahren gemäss Anspruch 1, worin wasserfreies Natrium-, Kalium-, Lithium- oder Ammoniumsulfat, bevorzugt wasserfreies Natriumsulfat verwendet wird.

3. Verfahren gemäss Anspruch 1, worin Hydroxylammoniumsulfat verwendet wird.

4. Verfahren gemäss Anspruch 1, worin ein wasserfreies anorganisches Sulfat verwendet wird, dessen Anteile mit einer Komgrösse ≤ 50 µm mindestens 1 Gew.-%, bevorzugt von 2 bis 20 Gew.-%, besonders bevorzugt von 5 bis 15 Gew.-% der Gesamtmenge wasserfreien Sulfats darstellen.

5. Verfahren gemäss Anspruch 1, worin zusätzlich ein inertes Verdünnungsmittel mit Schmelzpunkt ≤ 70°C, Siedepunkt ≥ 150°C / 1 bar und Dipolmoment µ ≤ 2×10⁻¹⁸ esu, ausgenommen Carbonsäuren, heteroaromatische basische Stickstoffverbindungen, Schwefelverbindungen und stark polare Verbindungen, gegebenenfalls in Mengen bis 150 Gew.-%, bevorzugt bis 50 Gew.-%, bezogen auf den eingesetzten Aldehyd, zugegeben wird.

6. Verfahren gemäss Anspruch 1, worin R₁, R₂ und R₃ unabhängig voneinander Wasserstoff, Halogen, C₁-C₄-Alkyl, C₁-C₄-Alkoxy, Di(C₁-C₄-alkyl)amino, R₄-C₆-C₁₀-Aryl, R₄-C₆-C₁₀-Aryloxy oder R₄-Di(C₆-C₁₀-aryl)amino, und R₄ Wasserstoff, Halogen, C₁-C₂-Alkyl, C₁-C₂-Alkoxy oder Di(C₁-C₂-alkyl)amino bedeuten.

7. Verfahren gemäss Anspruch 1, worin
R₁ Wasserstoff, Chlor, Methyl, Methoxy, tert.-Butyl oder Phenyl, bevorzugt 4-Methyl, 4-tert.-Butyl oder 4-Phenyl, besonders bevorzugt 4-Phenyl,
R₂ und R₃ entweder je Wasserstoff, oder beide zusammen ω-Buta-1,3-dienylen, und
R₄ Wasserstoff bedeuten.

8. Verfahren gemäss Anspruch 1, worin das Hydroxylammoniumsalz in Mengen von 0,505 bis 0,58 Mol, und das wasserfreie Sulfat in Mengen von 2 bis 12 Mol, je bezogen auf 1 Mol Aldehyd, eingesetzt werden.

9. Verfahren gemäss Anspruch 1, worin anschliessend das Nitril der Formel (I) durch direkte Destillation aus dem Reaktionsgemisch isoliert wird.

## Claims

1. A process for preparing a nitrile of the formula (I) 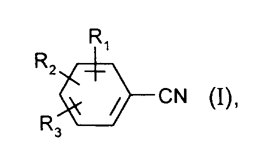
in which R₁, R₂ und R₃ independently of one another are hydrogen, halogen, cyano, hydroxyl, carboxyl, C₁-C₁₈alkyl, C₁-C₁₈alkoxy, C₁-C₁₈alkylthio, C₁-C₁₈alkylsulfonyl, C₁-C₁₈alkylamino, di(C₁-C₁₈alkyl)amino, R₄-C₆-C₁₀aryl, R₄-C₆-C₁₀aryloxy, R₄-C₆-C₁₀arylthio, R₄-C₆-C₁₀arylsulfonyl or R₄-C₆-C₁₀arylamino,
or, where R₂ and R₃ are ortho to one another, R₂ and R₃ together form a saturated or mono- or di-unsaturated, 4-membered carbon bridge which is substituted with a radical R₄,
and R₄ is hydrogen, halogen, cyano, hydroxyl, carboxyl, C₁-C₁₈alkyl, C₁-C₁₈alkoxy, C₁-C₁₈alkylthio, C₁-C₁₈alkylsulfonyl, C₁-C₁₈alkylamino or di(C₁-C₁₈alkyl)amino,
by reacting an aldehyde of the formula (II) 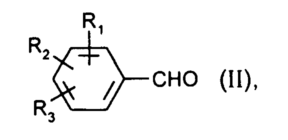 in which R₁, R₂ and R₃ are as defined above, with a hydroxylammonium salt followed by dehydration by heating to an elevated temperature, wherein the reaction takes place in the presence of an anhydrous inorganic sulfate and in the absence of diluents from the group consisting of carboxylic acids, strongly polar aprotic solvents, sulfur compounds and heteroaromatic basic nitrogen compounds.

2. A process according to claim 1, wherein anhydrous sodium sulfate, potassium sulfate, lithium sulfate or ammonium sulfate, preferably anhydrous sodium sulfate is used.

3. A process according to claim 1, wherein hydroxylammonium sulfate is used.

4. A process according to claim 1, wherein an anhydrous inorganic sulfate is used whose fractions having a particle size ≤ 50 µm constitute at least 1% by weight, preferably from 2 to 20% by weight, particularly preferably from 5 to 15% by weight of the overall quantity of anhydrous sulfate.

5. A process according to claim 1, wherein in addition an inert diluent having a melting point ≤ 70°C, a boiling ≥ 150°C / 1 bar and a dipole moment µ ≤ 2×10⁻¹⁸ esu, with the exception of carboxylic acids, heteroaromatic basic nitrogen compounds, sulfur compounds and strongly polar compounds, is added if desired in quantities of up to 150% by weight, preferably up to 50% by weight, based on the aldehyde employed.

6. A process according to claim 1, wherein R₁, R₂ and R₃ independently of one another are hydrogen, halogen, C₁-C₄alkyl, C₁-C₄alkoxy, di(C₁-C₄alkyl)amino, R₄-C₆-C₁₀aryl, R₄-C₆-C₁₀aryloxy or R₄-di(C₆-C₁₀aryl)amino, and R₄ is hydrogen, halogen, C₁-C₂alkyl, C₁-C₂alkoxy or di(C₁-C₂alkyl)amino.

7. A process according to claim 1, wherein
R₁ is hydrogen, chlorine, methyl, methoxy, tert-butyl or phenyl, preferably 4-methyl, 4-tert-butyl or 4-phenyl, particularly preferably 4-phenyl,
R₂ and R₃ either are each hydrogen, or both together are ω-buta-1,3-dienylene, and
R₄ is hydrogen.

8. A process according to claim 1, wherein the hydroxylammonium salt is employed in quantities of from 0.505 bis 0.58 mol, and the anhydrous sulfate is employed in quantities of from 2 to 12 mol, in each case per mole of aldehyde.

9. A process according to claim 1, in which the nitrile of the formula (I) is subsequently isolated from the reaction mixture by direct distillation.

## Revendications

1. Procédé pour la préparation d'un nitrile de formule (I) 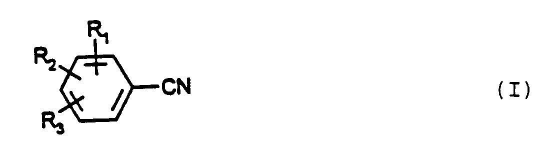
dans lequel R₁, R₂ et R₃ représentent, indépendamment l'un de l'autre, un atome d'hydrogène, un atome d'halogène, des groupes cyano, hydroxy, carboxy, alkyle en C₁-C₁₈, alkoxy en C₁-C₁₈, alkylthio en C₁-C₁₈, alkylsulfonyle en C₁-C₁₈, alkylamino en C₁-C₁₈, di(alkyl en C₁-C₁₈) amino, R₄-(aryle en C₆-C₁₀), R₄-(aryloxy en C₆-C₁₀), R₄-(aryl en C₆-C₁₀)thio, R₄-(aryl en C₆-C₁₀)-sulfonyle, R₄-(aryl en C₆-C₁₀)amino,
ou, dans le cas où R₂ et R₃ se trouvent en position ortho l'un par rapport à l'autre, R₂ et R₃ forment ensemble un pont carboné saturé ou à une ou deux insaturations, substitué en position 4 par un reste R₄,
et R₄ est un atome d'hydrogène, un atome d'halogène, des groupes cyano, carboxy, alkyle en C₁-C₁₈, alkoxy en C₁-C₁₈, (alkyl en C₁-C₁₈)thio, (alkyl en C₁-C₁₈)-sulfonyle, (alkyl en C₁-C₁₈)amino ou di(alkyle en C₁-C₁₈)-amino,
par réaction d'un aldéhyde de formule (II) 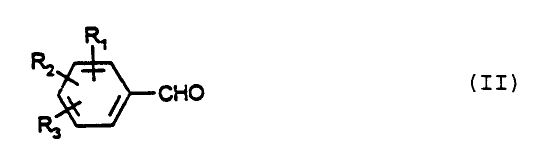 dans laquelle R₁, R₂ et R₃ possèdent la signification donnée ci-dessus, avec un sel d'hydroxylammonium, et suivie par une déshydratation en chauffant à une température élevée, caractérisé en ce que la réaction a lieu en présence d'un sulfate anhydre inorganique et en l'absence d'agents de dilution pris dans le groupe comportant des acides carboxyliques, des solvants aprotiques fortement polaires, des composés soufrés et des composés azotés basiques hétéroaromatiques.

2. Procédé selon la revendication 1, dans lequel on utilise du sulfate de sodium, de potassium, de lithium ou d'ammonium anhydre, de préférence du sulfate de sodium anhydre.

3. Procédé selon la revendication 1, dans lequel on utilise le sulfate d'hydroxylammonium.

4. Procédé selon la revendication 1, dans lequel on utilise un sulfate inorganique anhydre dont les taux d'une taille de grain ≤ 50 µm représentant d'au moins 1 % en poids, de préférence de 2 à 20 % en poids, de manière particulièrement préférée de 5 à 15 % en poids de la quantité totale du sulfate anhydre.

5. Procédé selon la revendication 1, dans lequel on ajoute en plus un diluant inerte avec un point de fusion ≤70°C, un point d'ébullition ≥150°C/1 bar et un moment dipolaire µ ≤2x10⁻¹⁸ esu, à l'exception des acides carboxyliques, des composés azotés basiques hétéroaromatiques, des composés soufrés et des composés fortement polaires, éventuellement dans des quantités jusqu'à 150 % en poids, de préférence jusqu'à 50 %, par rapport à l'aldéhyde utilisé.

6. Procédé selon la revendication 1, dans lequel R₁, R₂ et R₃ représentent, indépendamment l'un de l'autre, un atome d'hydrogène, un atome d'halogène, des groupes alkyle en C₁-C₄, alkoxy en C₁-C₄, di(alkyle en C₁-C₄)amino, R₄-(aryle en C₆-C₁₀), R₄-(aryloxy en C₆-C₁₀) ou R₄-di(aryl en C₆-C₁₀)amino, et R₄ représente un atome d'hydrogène, un atome d'halogène, des groupes alkyle en C₁-C₂, alkoxy en C₁-C₂ ou di(alkyl en C₁-C₂)amino.

7. Procédé selon la revendication 1, dans lequel
R₁ représente un atome d'hydrogène, un atome de chlore, des groupes méthyle, méthoxy, tert-butyle ou phényle, de préférence 4-méthyle, 4-tert-butyle ou 4-phényle, de manière particulièrement préférée, un groupe 4-phényle,
R₂ et R₃ représentent soit un atome d'hydrogène, soit les deux ensemble, le ω-buta-1,3-diénylène, et
R₄ représente un atome d'hydrogène.

8. Procédé selon la revendication 1, dans lequel on utilise le sel d'hydroxylammonium dans des quantités de 0,505 à 0,58 moles, et le sel anhydre dans des quantités de 2 à 12 moles, à chaque fois par rapport à 1 mole d'aldéhyde.

9. Procédé selon la revendication 1, dans lequel on isole ensuite le nitrile de formule (I) par distillation directe à partir du mélange réactionnel.
